(19) Europäisches Patentamt
European Patent Office
Office européen des brevets

(11) **EP 2 367 889 B1**

(12) **EUROPÄISCHE PATENTSCHRIFT**

(45) Veröffentlichungstag und Bekanntmachung des Hinweises auf die Patenterteilung:
**24.07.2013 Patentblatt 2013/30**

(21) Anmeldenummer: **10743035.7**

(22) Anmeldetag: **09.08.2010**

(51) Int Cl.:
**C09C 1/00** *(2006.01)*

(86) Internationale Anmeldenummer:
**PCT/EP2010/004864**

(87) Internationale Veröffentlichungsnummer:
**WO 2011/020569 (24.02.2011 Gazette 2011/08)**

(54) **HOCHGLÄNZENDE MEHRSCHICHTPERLGLANZPIGMENTE MIT ENGER GRÖSSENVERTEILUNG UND VERFAHREN ZU DEREN HERSTELLUNG**

HIGH-GLOSS MULTILAYER EFFECT PIGMENTS HAVING A NARROW SIZE DISTRIBUTION, AND METHOD FOR THE PRODUCTION THEREOF

PIGMENTS NACRÉS MULTICOUCHES HAUTE BRILLANCE À RÉPARTITION GRANULOMÉTRIQUE ÉTROITE ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.08.2009 DE 102009037932**

(43) Veröffentlichungstag der Anmeldung:
**28.09.2011 Patentblatt 2011/39**

(73) Patentinhaber: **Eckart GmbH**
**91235 Hartenstein (DE)**

(72) Erfinder:
• **SCHUMACHER, Dirk**
**91257 Pegnitz (DE)**
• **GRÜNER, Michael**
**91275 Auerbach (DE)**
• **KAUPP, Günter**
**91284 Neuhaus (DE)**

(74) Vertreter: **Walcher, Armin et al**
**Louis, Pöhlau, Lohrentz**
**Postfach 3055**
**90014 Nürnberg (DE)**

(56) Entgegenhaltungen:
**WO-A1-2009/103322     WO-A2-03/006558**
**WO-A2-2006/110359     US-A1- 2006 042 509**

## Beschreibung

Die vorliegende Erfindung betrifft Mehrschichtperlglanzpigmente mit verbesserten optischen Eigenschaften sowie ein Verfahren zur Herstellung derselben als auch deren Verwendung in kosmetischen Formulierungen, Kunststoffen, Folien, Textilien, keramischen Materialien, Gläsern. Beschichtungszusammensetzungen wie Farben, Druckfarben, Tinten, Lacken oder Pulverlacken.

Mehrschichtige Perlglanzpigmente zeichnen sich durch besonders *intensive* Interferenzfarben aus. Der klassische Aufbau dieser mehrschichtigen Perlglanzpigmente umfasst ein transparentes Substrat, welches mit mindestens einer Schichtenfolge umfassend eine hochbrechende, niedrigbrechende und hochbrechende Schicht beschichtet ist.

Im Vergleich zu Perlglanzpigmenten, die nur ein mit einer einzigen hochbrechenden Schicht beschichtetes Substrat aufweisen, zeigen Mehrschichtperlglanzpigmente in ihren Reflexionsspektren wesentlich engere Reflexionsbanden und wirken daher farbintensiver. Die dreischichtige Beschichtung wirkt für sich als Interferenzsystem, und daher sind bei diesen Mehrschichtperlglanzpigmenten im Gegensatz zu konventionellen Perlglanzpigmenten mit nur einer einzigen hochbrechenden Schicht die optischen Eigenschaften des Substrates weniger relevant. Weist die niedrigbrechende Schicht eine optische Schichtdicke von > 150 nm auf, so erhält man Mehrschichtperlglanzpigmente mit Farbflop. Ist die optische Schichtdicke unterhalb von 150 nm, so erhält man brillante Mehrschichtperlglanzpigmente mit intensiver Interferenzfarbe, jedoch ohne Farbflop.

Die JP 07246366 offenbart eine Vielzahl an Mehrschichtperlglanzpigmenten basierend auf C-Glas.

Die DE 19525503 A1 offenbart mehrschichtige Perlglanzpigmente mit einer transparenten niedrigbrechenden Schicht, gefolgt von einer selektiv oder nichtselektiv hochbrechenden Schicht.

Die DE 19953655 A1 offenbart mehrschichtige Perlglanzpigmente auf der Basis silikatischer Plättchen mit einer ersten Schicht, die aus $TiO_2$ besteht und in reduzierender Atmosphäre behandelt wurde. Hierbei entstehen farbige Schichten. Weiterhin weisen die Perlglanzpigmente eine farblose niedrigbrechende Schicht gefolgt von einer hochbrechenden Schicht, die Eisen(III) enthält, auf.

Die US 6875264 B2 offenbart Mehrschichtperlglanzpigmente mit einem Schichtaufbau, der einem non-quaterwave Stack entspricht.

Die EP 1474486 A2 offenbart Mehrschichtperlglanzpigmente basierend auf Glasflakesubstraten.

Die optischen Eigenschaften von Effektpigmenten können nach der WO 2006/110359 A2 durch eine geeignete Partikelgrößenverteilung beeinflusst werden. Die hier beschriebenen klassierten mit einer einzigen Metalloxidschicht beschichteten Glasflakes weisen einen $D_{10}$ von wenigstens 9,5 $\mu$m, vorzugsweise von 9,5 $\mu$m, auf. Nachteilig ist, dass die Pigmente einen Größenbereich mit einem $D_{10}$-Wert von maximal 85 $\mu$m, vorzugsweise von etwa 45 $\mu$m, aufzuweisen haben.

Die US 2006/0042509 A1 offenbart Perlglanzpigmente mit einem Metalloxid-haltigen plättchenförmigen Substrat und einer ersten und zweiten Schutzschicht, wobei die erste Schutzschicht Ceroxid und/oder Cerhydroxid umfasst und eine zweite Schutzschicht aus $SiO_2$, auf die eine organisch-chemische Nachbeschichtung aufgebracht ist.

WO 2009/103322 A1 betrifft Effektpigmente, umfassend künstliche plättchenförmige Substrate, die mindestens eine optisch wirksame Beschichtung aufweisen, wobei die Effektpigmente eine Volumen-gemittelte Größensummendurchgangsverteilungskurve mit den Kennzahlen $D_{10}$, $D_{50}$ und $D_{90}$ aufweisen, wobei diese Größensummendurchgangsverteilungskurve einen Span $\Delta D$ von 0,7 - 1,4 hat. Der Span $\Delta D$ berechnet sich gemäß der folgenden Formel: $\Delta D = (D_{90} - D_{10}) / D_{50}$. Die mittlere Dicke der künstlichen plättchenförmigen Substrate liegt in einem Bereich von 500 bis 200 nm.

Die WO 03/006558 A2 offenbart Mehrschichteffektpigmente auf Glasplättchen, wobei die Glasplättchen mit alternierenden Schichten mit hohem und niedrigem Brechungsindex beschichtet sind und die Glasplättchen wenigstens drei Schichten aufweisen.

Im Stand der Technik sind diverse mehrschichtige Perlglanzpigmente offenbart, die über ansprechende optische Eigenschaften verfügen. Dennoch besteht weiterhin ein Bedarf an verbesserten Produkten.

Aufgabe der vorliegenden Erfindung ist es, mehrschichtige Perlglanzpigmente mit verbessertem Glanz bereit zu stellen. Die mehrschichtigen Perlglanzpigmente sollten weiterhin ein höheres Chroma und möglichst einen höheren Farbflop als die mehrschichtigen Perlglanzpigmente aus dem Stand der Technik aufweisen.

Eine weitere Aufgabe besteht darin, ein einfaches Verfahren zur Bereitstellung der erfindungsgemäßen mehrschichtigen Perlglanzpigmente aufzufinden.

Die Aufgabe wird gelöst durch Bereitstellung von Mehrschichtperlglanzpigmenten basierend auf plättchenförmigen transparenten Substraten mit optisch wirksamer Beschichtung, wobei die optisch wirksame Beschichtung wenigstens

a) eine nicht absorbierende hochbrechende Schicht A mit einem Brechungsindex n $\geq$ 1,8

b) eine niedrigbrechende Schicht B mit einem Brechungsindex n < 1,8 und mit einer optischen Schichtdicke > 150 nm

c) eine absorbierende hochbrechende Schicht C mit einem Brechungsindex n $\geq$ 1,8 sowie

d) optional eine äußere Schutzschicht D

umfasst, und dass die Mehrschichtperlglanzpigmente eine Summenhäufigkeitsverteilung der volumengemittelten Größenverteilungsfunktion mit den Kennzahlen $D_{10}$, $D_{50}$, $D_{90}$ und einem Span $\Delta D$ von 0,7 -1,4 aufweisen, wobei der Span $\Delta D$ gemäß der Formel $\Delta D = (D_{90} - D_{90})/ D_{50}$ berechnet ist.

Bevorzugte Weiterbildungen sind in den Unteransprüchen 2 bis 11 angegeben.

Die Aufgabe wird weiterhin gelöst durch Bereitstellung eines Verfahrens zum Herstellen der Mehrschichtperlglanzpigmente, welches folgende Schritte umfasst:

(i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 - 1,4 aufweisen, wobei der Span $\Delta D$ gemäß der Formel $\Delta D = (D_{90} - D_{10})/D_{50}$ definiert ist,

(ii) Aufbringen wenigstens der Schichten A bis C auf die plättchenförmigen Substrate sowie optional wenigstens einer Schicht D,

oder

(iii) Aufbringen wenigstens der Schichten A bis C auf die plättchenförmigen transparenten Substrate sowie optional wenigstens einer Schicht D,

(iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einen Span $\Delta D$ in einem Bereich von 0,7 - 1,4 aufweisen, wobei der Span $\Delta D$ gemäß der Formel $\Delta D = (D_{90} - D_{10})/D_{50}$ definiert ist.

Vorzugsweise erfolgt das Beschichten der plättchenförmigen transparenten Substrate in Schritt (ii) nach dem Größenklassieren in Schritt (i).

Des Weiteren ist Gegenstand der Erfindung die Verwendung der erfindungsgemäßen Mehrschichtperlglanzpigmente in kosmetischen Formulierungen, Kunststoffen, Folien, keramischen Materialien, Gläsern und Beschichtungszusammensetzungen, wie Farben, Druckfarben, Tinten, Lacken und Pulverlacken. Gegenstand der Erfindung sind mithin Zubereitungen, die die erfindungsgemäßen Mehrschichtperlglanzpigmente enthalten. Die Erfindung ist auch auf Gegenstände gerichtet, die mit den erfindungsgemäßen Mehrschichtperlglanzpigmenten versehen, beispielsweise lackiert oder bedruckt, sind. Somit sind lackierte Gegenstände, wie Karosserien, Fassadenelemente, etc. oder bedruckte Gegenstände, wie Papier, Pappe, Folien, Textilien etc., ebenfalls Bestandteil der vorliegenden Erfindung.

Die Erfinder haben überraschend festgestellt, dass die erfindungsgemäßen Mehrschichtperlglanzpigmente, d.h. Perlglanzpigmente mit der angegebenen Schichtenanordnung und einem Span $\Delta D = (D_{90} - D_{10})/D_{50}$ im Bereich von 0,7 bis 1,4, einen außerordentlich starken Glanz aufweisen. Gemäß einer weiteren Variante der Erfindung weisen die erfindungsgemäßen Mehrschichtperlglanzpigmente zusätzlich auch ein hohes Chroma und einen hohen Farbflop auf.

Die Bereitstellung von Mehrschichtperlglanzpigmenten mit starkem Glanz, und vorzugsweise zugleich auch hohem Chroma und Farbflop, ist im Stand der Technik bislang nicht möglich gewesen.

Überraschenderweise wurde gefunden, dass Mehrschichtperlglanzpigmente basierend auf Substraten mit engem Span in der Größenverteilung, die mit wenigstens dem optisch wirksamen Dreischichtaufbau beschichtet sind, einen deutlich höheren Glanz sowie höheres Chroma gegenüber Mehrschichtperlglanzpigmenten mit einem breiteren Span, wie dies im Stand der Technik der Fall ist, aufweisen.

Dass der Span $\Delta D$, wie in Anspruch 1 definiert, einen Einfluss auf den Glanz haben könnte, ist im Hinblick auf den veröffentlichten Stand der Technik unerwartet. Somit ermöglicht die vorliegende Erfindung die Bereitstellung von Mehrschichtperlglanzpigmenten, die äußerst ansprechende optische Eigenschaften aufweisen.

Im Stand der Technik wurde bislang der Span $\Delta D$ nicht als ein wesentliches Merkmal erkannt. Mithin weisen herkömmliche Mehrschichtperlglanzpigmente einen breiten Span auf.

Zur Charakterisierung der Teilchengrößenverteilung wird erfindungsgemäß der Span $\Delta D$, definiert als $\Delta D = (D_{90} - D_{10})/D_{50}$, verwendet. Je kleiner der Span ist, desto enger ist die Teilchengrößenverteilung.

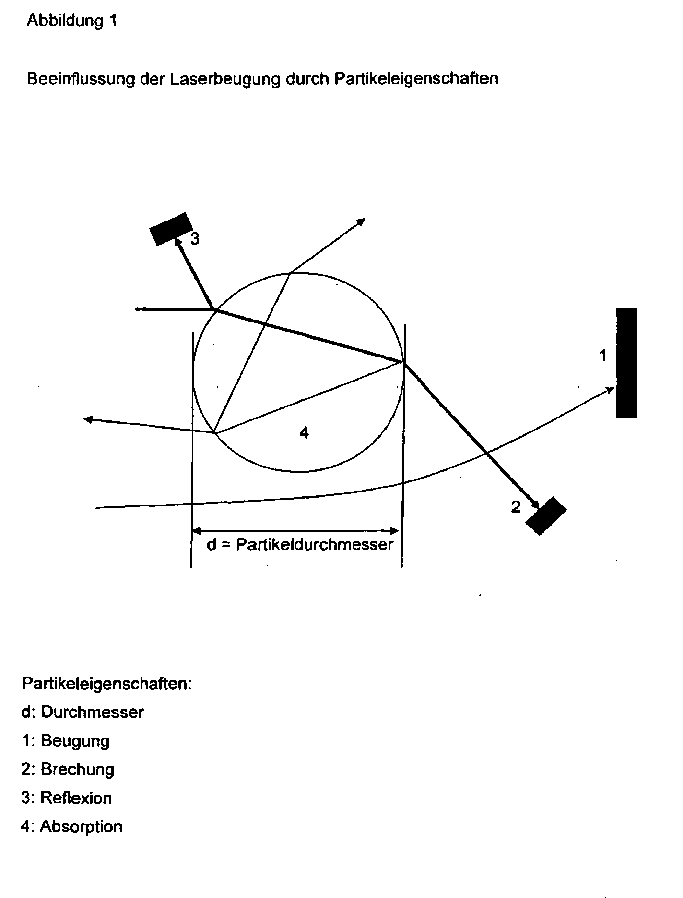

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der Mehrschichtperlglanzpigmente einen Durchmesser aufweisen, der gleich oder kleiner als der jeweils angegebene Wert ist. Hierbei wird die Größenverteilungskurve mit einem Gerät der Fa. Malvern (Gerät: MALVERN Mastersizer 2000) gemäß Herstellerangaben bestimmt. Die Auswertung der Streulichtsignale erfolgte dabei nach der Mie-Theorie, welche auch Brechungs- und Absorptionsverhalten der Partikel beinhaltet (Abbildung 1)

Die erfindungsgemäßen Mehrschichtperlglanzpigmente besitzen einen Span $\Delta D$ in einem Bereich von 0,7 bis 1,4, vorzugsweise in einem Bereich von 0,7 bis 1,3, weiterhin bevorzugt in einem Bereich von 0,8 bis 1,2, besonders bevorzugt in einem Bereich von 0,8 bis 1,1. Bei weiterhin bevorzugten Ausführungsformen liegt der Span $\Delta D$ in einem Bereich von 0,85 bis 1,05.

Weisen die Mehrschichtperlglanzpigmente einen Span $\Delta D$ oberhalb von 1,4 auf, so werden keine hochglänzenden Mehrschichtperlglanzpigmente erhalten. Mehrschichtperlglanzpigmente unterhalb eines Spans ΔD von 0,7 können im Rahmen der üblichen Methoden nur sehr aufwändig und damit nicht mehr wirtschaftlich hergestellt werden.

Der Span ΔD des zu beschichtenden, plättchenförmigen transparenten Substrats entspricht im Wesentlichen dem des erfindungsgemäßen Mehrschichtperlglanzpigments und liegt bei ≤ 1,4, vorzugsweise ≤ 1,3, weiter bevorzugt ≤ 1,2, besonders bevorzugt ≤ 1,1 und ganz besonders bevorzugt bei ≤ 1,05.

Die erfindungsgemäßen Mehrschichtperlglanzpigmente können eine beliebige mittlere Teilchengröße ($D_{50}$) aufweisen. Die $D_{50}$-Werte der erfindungsgemäßen Mehrschichtperlglanzpigmente liegen vorzugsweise in einem Bereich von 3 bis 350 μm. Bevorzugt weisen die erfindungsgemäßen Mehrschichtperlglanzpigmente einen $D_{50}$-Wert aus einem Bereich von 3 bis 15 μm oder aus einem Bereich von 10 bis 35 μm oder aus einem Bereich von 25 bis 45 μm oder aus einem Bereich von 30 bis 65 μm oder aus einem Bereich von 40 bis 140 μm oder aus einem Bereich von 135 bis 250 μm auf.

Die $D_{10}$-Werte der erfindungsgemäßen Mehrschichtperlglanzpigmente umfassen vorzugsweise einen Bereich von 1 bis 120 μm. Bevorzugt weisen die erfindungsgemäßen Mehrschichtperlglanzpigmente die in Tabelle 1 angegebenen Kombinationen von $D_{10}$-, $D_{50}$- und $D_{90}$-Werten auf. Hierbei werden die $D_{10}$-, $D_{50}$- und $D_{90}$-Werte der Tabelle 1 nur in der Art kombiniert, dass sich ein Span ΔD aus einem Bereich von 0,7 bis 1,4, vorzugsweise aus einem Bereich von 0,7 bis 1,3, weiterhin bevorzugt aus einem Bereich von 0,8 bis 1,2, besonders bevorzugt aus einem Bereich von 0,8 bis 1,1 und ganz besonders bevorzugt aus einem Bereich von 0,85 bis 1,05 ergibt. Kombinationen von $D_{10}$-, $D_{50}$- und $D_{90}$-Werten, die zu einem Span ΔD führen, der nicht im Bereich von 0,7 bis 1,4 liegt, sind keine erfindungsgemäßen Ausführungsformen.

Tabelle 1: Bevorzugte Kombinationen von Bereichen der $D_{10}$-, $D_{50}$- und $D_{90}$-Werte

| $D_{10}$ (μm) | $D_{50}$ (μm) | $D_{90}$ (μm) |
|---|---|---|
| 1 - 5 | 3 - 15 | 8 - 25 |
| 5 - 25 | 10 - 35 | 20 - 45 |
| 10 - 30 | 25 - 45 | 40 - 70 |
| 20 - 45 | 30 - 65 | 70 - 110 |
| 25 - 65 | 40 - 140 | 120 - 180 |
| 75 - 110 | 135 - 250 | 400 - 490 |

Dabei hat sich überraschenderweise gezeigt, dass die Größe der Mehrschichtperlglanzpigmente, charakterisiert durch den $D_{50}$- Wert, nicht entscheidend ist, sondern dass der Span ΔD = ($D_{90}$ - $D_{10}$)/ $D_{50}$ in einem engen Bereich von 0,7 bis 1,4 liegt. Dabei können beispielsweise die $D_{50}$-Werte der Mehrschichtperlglanzpigmente bei 15, 20, 25, 30 μm oder auch bei 50, 80, 100, 150, 200, 250, 300 oder 350 μm liegen.

Es hat sich gezeigt, dass überraschenderweise auch bei einer Mehrzahl von größeren Mehrschichtperlglanzpigmenten glanzstarke und vorzugsweise hochchromatische Mehrschichtperlglanzpigmente erhalten werden, wenn der Span ΔD in einem Bereich von 0,7 bis 1,4 liegt.

Gemäß einer weiteren bevorzugten Ausführungsform der Erfindung weisen die Mehrschichtperlglanzpigmente wenigstens zwei Interferenzfarben auf. Bei dieser Variante der Erfindung kommt es mithin zu einem winkelabhängigen Wechsel zwischen zwei oder mehr Interferenzfarben. Bei Änderung des Betrachtungswinkels ändert sich mithin die Interfererizfarbe, beispielsweise von rot nach grün oder von blau nach gelb. Die erfindungsgemäßen Mehrschichtperlglanzpigmente gemäß dieser Variante weisen mithin einen winkelabhängigen Interferenzfarbenwechsel auf und können auch als goniochromatische Mehrschichtperlglanzpigmente bezeichnet werden.

Der Übergang zwischen Mehrschichtperlglanzpigmenten mit keinem, schwachem und intensivem Farbflop in Abhängigkeit von der optischen Schichtdicke der niedrigbrechenden Schicht B verläuft fließend. Mit zunehmender optischer Schichtdicke der niedrigbrechenden Schicht B werden ab 150 nm zunächst Mehrschichtperlglanzpigmente erhalten, die nur einen schwachen Farbflop aufweisen, welcher letztendlich mit immer weiter steigender optischen Schichtdicke von Schicht B in einen intensiven Farbflop übergeht. Typischerweise erstreckt sich ein intensiver Farbflop über mehrere Quadranten im CIELab-Farbkoordinatensystem.

Gemäß einer weiteren bevorzugten Ausführungsform der Erfindung weisen die erfindungsgemäßen Mehrschichtperlglanzpigmente keine silberne Interferenzfarbe auf. Die erfindungsgemäßen Mehrschichtperlglanzpigmente zeichnen sich dadurch aus, dass diese vorzugsweise bei einem glanznahen Betrachtungswinkel farbig, jedoch nicht silberfarben sind. Die erfindungsgemäßen Mehrschichtperlglanzpigmente unterscheiden sich mithin signifikant von silberfarbenen Interferenz- und Metalleffektpigmenten, insbesondere von silberfarbenen Aluminiumeffektpigmenten.

Vorzugsweise weisen die erfindungsgemäßen Mehrschichtperlglanzpigmente wenigstens eine Interferenzfarbe auf, die aus der Gruppe ausgewählt wird, die aus gelb, violett, blau, rot, grün und Abstufungen davon besteht, jedoch keine silberne Interferenzfarbe umfasst. Die jeweilige Interferenzfarbe kann dabei von dunkel bis hell reichen.

Die erfindungsgemäßen Mehrschichtperlglanzpigmente zeichnen sich durch einen starken Glanz aus sowie vorzugsweise zugleich durch ein hohes Chroma $C^*_{15} > 20$ bei einem Beobachtungswinkel nahe dem Glanzwinkel.

Die Chromawerte werden dabei anhand von folgenden Applikationen ermittelt: Ein Nitrocelluloselack (Dr. Renger Erco Bronzemischlack 2615e; Fa. Morton), der 6 Gew.-% an Mehrschichtperlglanzpigmenten enthält, wobei sich die Gew.-% Angabe auf das Gesamtgewicht des Lackes bezieht, wird je nach $D_{50}$-Wert in einer Nassfilmdicke gemäß Tabelle 2 auf BYK-Gardner Schwarz-Weiß-Rakelkarten (Byko-Chart 2853) aufgetragen und nachfolgend bei Raumtemperatur getrocknet. An diesen Rakelkarten werden dann mit einem BYK-MAC (Fa. Byk-Gardner) farbmetrische Auswertungen vorgenommen, wobei auf dem schwarzen Untergrund der Rakelkarte vermessen wird. Der Einfallswinkel beträgt 45° und herangezogen wird der Chromawert bei einem Beobachtungswinkel von 15°.

Tabelle 2: Nassfilmdicke in Abhängigkeit vom $D_{50}$-Wert der Mehrschichtperlglanzpigmente

| $D_{50}$-Wert | Spiralrakel |
|---|---|
| <40 $\mu$m | 36 $\mu$m |
| 40 $\mu$m - 85 $\mu$m | 76 $\mu$m |
| > 85 $\mu$m | 100 $\mu$m |

Gemäß einer bevorzugten Variante der Erfindung beträgt das Chroma $C^*_{15}$ der erfindungsgemäßen Mehrschichtperlglanzpigmente wenigstens 22, vorzugsweise wenigstens 24, noch weiter bevorzugt wenigstens 25. Als sehr geeignet hat sich ein Chroma in einem Bereich von 24 bis 50 erwiesen.

Die erfindungsgemäßen Mehrschichtperlglanzpigmente werden hergestellt, indem die plättchenförmigen Substrate mit wenigstens einer optisch wirksamen Beschichtung versehen werden, welche

    a) eine nicht absorbierende hochbrechende Schicht A mit einem Brechungsindex n $\geq$ 1,8
    b) eine niedrigbrechende Schicht B mit einem Brechungsindex n < 1,8 und mit einer optischen Schichtdicke > 150 nm
    c) eine absorbierende hochbrechende Schicht C mit einem Brechungsindex n $\geq$ 1,8 sowie
    d) optional eine äußere Schutzschicht D

umfasst. Die Schichten A und B bzw. B und C können unter der äußeren Schutzschicht D mehrfach aufgebracht werden. Bevorzugt werden immer alternierend hoch- und niedrigbrechende Schichten auf dem Substrat aufgebracht. Besonders bevorzugt wird das plättchenförmige transparente Substrat nur einmal mit den Schichten A bis C, optional D beschichtet.

Erfindungsgemäß ist die Schicht A in der Schichtenanordnung innenliegend, d.h. dem plättchenförmigen transparenten Substrat zugewandt, die Schicht B zwischen der Schicht A und der Schicht C liegend, und die Schicht C, bezogen auf das plättchenförmige transparente Substrat, in der Schichtenanordnung außenliegend.

Zwischen dem plättchenförmigen transparenten Substrat und der Schicht A können eine oder mehrere weitere, vorzugsweise im Wesentlichen transparente Schichten, angeordnet sein. Gemäß einer bevorzugten Weiterbildung ist die Schicht A direkt auf dem plättchenförmigen transparenten Substrat aufgebracht.

Zwischen der Schicht A und der Schicht B sowie zwischen der Schicht B und der Schicht C können unabhängig voneinander eine oder mehrere weitere, vorzugsweise im Wesentlichen transparente Schichten, angeordnet sein. Gemäß einer bevorzugten Weiterbildung ist die Schicht B direkt auf der Schicht A aufgebracht. Gemäß einer weiteren bevorzugten Weiterbildung ist die Schicht C direkt auf der Schicht B aufgebracht.

Äußerst bevorzugt ist die Schicht A unmittelbar auf dem plättchenförmigen transparenten Substrat, die Schicht B unmittelbar auf der Schicht A, die Schicht C unmittelbar auf der Schicht B sowie optional die Schicht D unmittelbar auf der Schicht C aufgebracht.

Als optisch wirksame Schichten oder Beschichtungen werden vorzugsweise Schichten aufgebracht, die Metalloxide, Metalloxidhydrate, Metallhydroxide, Metallsuboxide, Metalle, Metallfluoride, Metalloxyhalide, Metallchalcogenide, Metallnitride, Metalloxynitride, Metallsulfide, Metallcarbide oder Mischungen davon umfassen. Gemäß einer bevorzugten Variante bestehen die optisch wirksamen Schichten oder Beschichtungen aus den vorstehend genannten Materialien, wobei Metalloxide besonders bevorzugt sind.

Die Begriffe Schichten oder Beschichtungen werden im Sinne dieser Erfindung austauschbar verwendet, sofern nicht anders angegeben.

Bei den erfindungsgemäßen Mehrschichtpigmenten werden die optischen Wirkungen durch den Schichtenaufbau mit den Schichten A bis C hervorgerufen, an dem einfallendes Licht durch physikalische Effekte wie Reflexion, Interferenz, Absorption, Lichtbrechung, etc. die wahrnehmbaren Farbeffekte erzeugt.

Die optische Dicke der nichtmetallischen Schichten mit hoher und niedriger Brechzahl bestimmt die optischen Eigenschaften der Mehrschichtperlglanzpigmente. Die Anzahl und Dicke der Schichten können in Abhängigkeit vom gewünschten Effekt und vom verwendeten Substrat eingestellt werden. Wenn n die Brechzahl einer Schicht und d ihre Dicke ist, ergibt sich die Interferenzfarbe, in der eine dünne Schicht erscheint, aus dem Produkt von n und d, d.h. der optischen Dicke. Die bei Normallichteinfall im reflektierenden Licht entstehenden Farben eines solchen Filmes ergeben sich aus einer Verstärkung des Lichtes der Wellenlänge

$$\lambda = \frac{4}{2N-1} \cdot nd$$

und durch Schwächung von Licht der Wellenlänge

$$\lambda = \frac{2}{N} \cdot nd \, ,$$

wobei N eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz.

Bei mehrschichtigen Pigmenten wird die Interferenzfarbe durch die Verstärkung bestimmter Wellenlängen bestimmt und, wenn mehrere Schichten in einem vielschichtigen Pigment die gleiche optische Dicke besitzen, wird die Farbe des reflektierenden Lichtes mit zunehmender Zahl der Schichten intensiver ("Quaterwave-stack"). Darüber hinaus kann durch geeignete Wahl der Schichtdicke der niedrigbrechenden Schicht B eine besonders starke Variation der Farbe in Abhängigkeit vom Betrachtungswinkel erreicht werden. Es kann sich so ein ausgeprägter Farbflop ausbilden.

Die einzelnen Schichten der erfindungsgemäßen Mehrschichtperlglanzpigmente können jeweils als $\lambda$/4-Schichten ausgebildet sein. Es hat sich jedoch überraschend herausgestellt, dass es nicht erforderlich ist, dass die Schichten als $\lambda$/4-Schichten ausgebildet sein müssen, um glanzstarke Mehrschichtperlglanzpigmente zu erhalten. Der entscheidende Parameter ist der Span der Größenverteilung, um glanzstarke Mehrschichtperlglanzpigmente zu erhalten.

Die nicht absorbierende hochbrechende Schicht A weist einen Brechungsindex n $\geq$ 1,8 auf. Bevorzugt ist der Brechungsindex n $\geq$ 1,9 und besonders bevorzugt n $\geq$ 2,0.

Zu den hochbrechenden nicht absorbierenden Materialien zählen beispielsweise

- ■ Metalloxide wie Titandioxid, Zirkoniumdioxid, Zinkoxid, Zinndioxid, Antimonoxid und deren Gemische,
- ■ Metallhydroxide,
- ■ Metalloxidhydrate,
- ■ Metallsulfide wie Zinksulfid,
- ■ Metalloxyhalide wie Bismutoxychlorid.

Die nicht absorbierende hochbrechende Schicht A umfasst bevorzugt Metalloxidschichten. Bevorzugte Metalloxidschichten umfassen Titanoxid, insbesondere $TiO_2$, Zirkoniumoxid, vorzugsweise $ZrO_2$, Zinkoxid, vorzugsweise ZnO, Zinnoxid, vorzugsweise $SnO_2$, oder Mischungen hieraus. Besonders bevorzugt ist die nicht absorbierende hochbrechende Schicht A eine Schicht, die $TiO_2$ umfasst oder weitgehend hieraus besteht. Das $TiO_2$ kann in der Anatas-, Pseudobrookit- oder der Rutilmodifikation vorliegen. Bevorzugt liegt das $TiO_2$ in der Anatas und/oder der Rutilmodifikation vor und ganz besonders bevorzugt in der Rutilmodifikation. Um Titanoxid, vorzugsweise $TiO_2$, in der Rutilform aufzubringen kann unterhalb der Titanoxidschicht zunächst Zinnoxid, vorzugsweise $SnO_2$, aufgebracht werden. Das Zinnoxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann.

Die optische Schichtdicke der Schicht A liegt bevorzugt in einem Bereich von 30 bis 900 nm, besonders bevorzugt bei 40 bis 880 nm und besonders bevorzugt bei 50 bis 850 nm.

Bei den in dieser Anmeldung angegebenen Schichtdicken handelt es sich - sofern nicht anders angegeben - um die optischen Schichtdicken. Unter einer optischen Schichtdicke wird erfindungsgemäß das Produkt aus geometrischer Schichtdicke und dem Brechungsindex des die Schicht aufbauenden Materials verstanden. Als Wert für den Brechungsindex des jeweiligen Materials wird der aus der Literatur jeweils bekannte Wert verwendet.

Die geometrische Schichtdicke wird erfindungsgemäß anhand von Querschliffaufnahmen von Lacken, in denen planparallel zum Untergrund orientierte Mehrschichtperlglanzpigmente vorliegen, mittels REM bestimmt.

Unterhalb einer optischen Schichtdicke von 30 nm ist der Interferenzeffekt zu wenig ausgeprägt. Optische Schichtdicken über 900 nm werden insgesamt zu dick und führen zu einer zu hohen Gesamtdicke des Mehrschichtperlglanzpigments. Derartige Mehrschichtperlglanzpigmente orientieren sich schlecht im Anwendungsmedium und weisen daher Nachteile auf.

Die niedrigbrechende Schicht B hat in der Regel einen Brechungsindex n < 1,8, bevorzugt < 1,7 und ganz besonders bevorzugt < 1,6.

Als niedrigbrechende Schicht B eignen sich nicht absorbierende Materialien. Hierzu zählen beispielsweise

- Metalloxide wie Siliziumdioxid, Aluminiumoxid, Boroxid,
- Metalloxidhydrate wie Siliziumoxidhydrat, Aluminiumoxidhydrat,
- Metallfluoride wie Magnesiumfluorid,
- $MgSiO_3$.

Gegebenenfalls kann die niedrigbrechende Metalloxidschicht Alkali- und Erdalkalioxide als Bestandteile enthalten.

Vorzugsweise umfasst die niedrigbrechende Schicht B Siliziumdioxid. In einer Ausführungsform besteht die niedrigbrechende Schicht B aus Siliziumdioxid.

Da die Schicht B im Wesentlichen den Wechsel der Interferenzfarben der erfindungsgemäßen Mehrschichtperlglanzpigmente bestimmt, weist die Schicht B für Mehrschichtperlglanzpigmente, die ein besonders ausgeprägtes Farbenspiel zeigen und daher auch bevorzugt sind, und die auf der Schicht A nur ein Schichtpaket (B) + (C) aufweisen, eine optische Mindestschichtdicke von > 150 nm auf. Die optische Schichtdicke der Schicht B liegt bevorzugt in einem Bereich von > 150 nm bis 500 nm, besonders bevorzugt in einem Bereich von 180 nm bis 480 nm und ganz besonders bevorzugt in einem Bereich von 220 nm bis 450 nm. Derartige Pigmente weisen einen ausgeprägten Farbflop auf.

Die absorbierende hochbrechende Schicht C kann gemäß einer Ausführungsform selektiv absorbierend oder nicht selektiv absorbierend sein.

Als hochbrechende, für die Schicht C geeignete Materialien sind z.B. Materialien wie nichtselektiv absorbierende Metalle, Metalloxide, Metallsulfide oder deren Mischungen, die auch selektiv absorbierende Metalloxide, vorzugsweise in untergeordneter Menge, enthalten können, sowie selektiv absorbierende Materialien wie insbesondere Metalloxide zu nennen, die in der Regel jeweils einen Brechungsindex n ≥ 1,8, bevorzugt n ≥ 1,9 und ganz besonders bevorzugt n ≥ 2,0 haben.

Beispiele für hochbrechende nichtselektiv absorbierende Materialien sind u.a.

- Metalle wie Molybdän, Eisen, Wolfram, Chrom, Cobalt, Nickel, Silber, Palladium, Platin, deren Gemische oder Legierungen,
- Metalloxide wie Magnetit $Fe_3O_4$, Cobaltoxid (CoO und/ oder $Co_3O_4$), Vanadiumoxid ($VO_2$ und/ oder $V_2O_3$) sowie auch Mischungen dieser Oxide mit Metallen, insbesondere Magnetit und (metallischem) Eisen,
- Eisentitanante wie limenit,
- Metallsulfide wie Moybdänsulfid, Eisensulfid, Wolframsulfid, Chromsulfid, Colbaltsulfid, Nickelsulfid, Silbersulfid, Zinnsulfid, Gemische dieser Sulfide, Gemische dieser Sulfide mit dem jeweiligen Metall, wie $MoS_2$ und Mo, und Gemische mit Oxiden des jeweiligen Metalls, wie $MoS_2$ und Molybdänoxide,
- nicht absorbierende farblose hochbrechende Schichten wie Titandioxid oder Zirkoniumdioxid, in die nichtselektiv absorbierendes Material (z.B. Kohlenstoff) eingebaut ist oder die damit beschichtet sind.

Als hochbrechende selektiv absorbierende Materialien eignen sich beispielsweise

- farbige Metalioxide oder Metalloxidhydrate wie Eisen(III)oxid (α- und/ oder γ-$Fe_2O_3$, rot), FeO(OH) (gelb), Chrom(III)oxid (grün), Titan(III)oxid ($Ti_2O_3$, blau), Vanadiumpentoxid (orange),
- farbige Nitride wie Titanoxynitride und Titannitrid ($TiO_xN_y$, TiN, blau), wobei die niederen Titanoxide und -nitride in der Regel im Gemisch mit Titandioxid vorliegen,
- Metallsulfide wie Cersulfid (rot),
- Eisentitanate wie Pseudobrookit (braunrot) und/ oder Pseudorutil (braunrot),
- Zinn-Antimonoxid Sn(Sb)$O_2$.
- nicht absorbierende farblose hochbrechende Materialien, z.B. Metalloxide wie Titandioxid und Zirkoniumdioxid, die mit selektiv absorbierenden Farbmitteln eingefärbt sind. Diese Einfärbung kann durch Einbau von Farbmitteln in die Metalloxidschicht, durch deren Dotierung mit selektiv absorbierenden Metallkationen oder farbigen Metalloxiden wie Eisen(III)oxid oder durch Überziehen der Metalloxidschicht mit einem ein Farbmittel enthaltenden Film erfolgen.

Besonders bevorzugt werden für Schicht C selektiv absorbierende Eisenoxide verwendet.

Die Schicht C kann auch Mischungen verschiedener selektiv und/ oder nicht selektiv absorbierender Komponenten, vorzugsweise Metalloxide, enthalten. Beispielsweise können die verschiedenen Komponenten, vorzugsweise Metalloxide, als homogene Mischung vorliegen. Es ist aber auch möglich, dass die eine Komponente in der anderen Komponente in Form einer Dotierung vorliegt.

Beispielsweise kann in der Schicht C eine nicht absorbierende Komponente, beispielsweise Titanoxid, vorzugsweise $TiO_2$, als Dotierung in einer selektiv absorbierenden Komponente, vorzugsweise $Fe_2O_3$, und/ oder einer nicht selektiv absorbierenden Komponente, beispielsweise $Fe_3O_4$, vorliegen. Alternativ kann auch eine selektiv absorbierende Komponente, beispielsweise $Fe_2O_3$, und/ oder eine nicht selektiv absorbierende Komponente, beispielsweise $Fe_3O_4$, als Dotierung in einer nicht absorbierenden Komponente, beispielsweise Titanoxid, vorzugsweise $TiO_2$, vorliegen.

Auch ist es selbstverständlich möglich, dass Gemische von mehr als zwei Komponenten, wie vorstehend veranschaulicht, in der Schicht C vorliegen.

Schließlich eignen sich als auch nichtselektiv absorbierende Materialien mit Absorptionskanten für die Schicht C insbesondere Metalle wie z.B. Aluminium.

Im Fall von Metallen liegen die geometrischen Schichtdicken der Schicht C in einem Bereich von 10 bis 50 nm, bevorzugt von 15 bis 30 nm: Die Schichtdicken müssen so eingestellt werden, dass die Schichten semitransparente Eigenschaften aufweisen. Die geometrischen Schichtdicken werden mithin in Abhängigkeit vom verwendeten Metall eingestellt, so dass die Schicht teil- oder semitransparent ist.

Die optische Schichtdicke der Schicht C liegt vorzugsweise in einem Bereich von 30 bis 900 nm, weiter bevorzugt in einem Bereich von 40 nm bis 880 nm, noch weiter bevorzugt in einem Bereich von 50 nm bis 850 nm.

Umfasst die Schicht C ein Metalloxid und/ oder ein Metallsulfid, so liegt die optische Schichtdicke dieser Schicht C vorzugsweise in einem Bereich von 30 nm bis 900 nm, bevorzugt in einem Bereich von 40 nm bis 880 nm und besonders bevorzugt in einem Bereich von 50 nm bis 850 nm.

Geeignete zu beschichtende plättchenförmige transparente Substrate sind nichtmetallische, natürliche oder synthetische plättchenförmige Substrate. Die Substrate sind vorzugsweise im Wesentlichen transparent, bevorzugt transparent, d.h. für sichtbares Licht sind sie zumindest teilweise durchlässig.

Gemäß einer bevorzugten Ausführungsform der Erfindung können die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen, Polymerplättchen, plättchenförmigem Bismuthoxychlorid, plättchenförmigen Substraten umfassend eine anorganisch-organische Mischschicht und deren Gemische, ausgewählt werden. Bevorzugt werden die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt. Besonders bevorzugt werden die plättchenförmigen transparenten Substrate aus der Gruppe, bestehend aus natürlichem Glimmer, synthetischem Glimmer, Glasflakes und deren Gemische, ausgewählt. Ganz besonders bevorzugt sind Glasflakes und synthetischer Glimmer und deren Gemische.

Natürlicher Glimmer besitzt im Unterschied zu synthetischen plättchenförmigen transparenten Substraten den Nachteil, dass Verunreinigungen durch eingelagerte Fremdionen den Farbton verändern können und dass *die* Oberfläche nicht ideal glatt ist, sondern Unregelmäßigkeiten, wie z.B. Stufen aufweisen kann.

Jedoch auch bei Verwendung von natürlichem Substrat hat sich überraschenderweise gezeigt, dass der Glanz einer Mehrzahl von Mehrschichtperlganzpigmenten gesteigert werden kann, wenn der Span $\Delta D$ in einem Bereich von 0,7 bis 1,4 liegt, verglichen mit einer Mehrzahl an herkömmlichen Mehrschichtperlglanzpigmenten mit breitem Span.

Synthetische Substrate wie beispielsweise Glasflakes oder synthetischer Glimmer dagegen weisen glatte Oberflächen, eine einheitliche Dicke innerhalb eines einzelnen Substratteilchens sowie scharfe Kanten auf. Somit bietet die Oberfläche nur wenig Streuzentren für einfallendes bzw. reflektiertes Licht und ermöglicht so nach Beschichtung höher glänzende Mehrschichtperlglanzpigmente als mit natürlichem Glimmer als Substrat. Als Glasflakes werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasflakes können beispielsweise eine Zusammensetzung entsprechend der Lehre der EP 1 980 594 B1 aufweisen.

Die mittlere geometrische Dicke der zu beschichtenden plättchenförmigen transparenten Substrate liegt in einem Bereich von 50 nm bis 5000 nm, bevorzugt in einem Bereich von 60 nm bis 3000 nm und besonders bevorzugt in einem Bereich von 70 nm bis 2000 nm. Bei einer Ausführungsform liegt die mittlere geometrische Dicke für Glasflakes als zu beschichtendes Substrat in einem Bereich von 750 nm bis 1500 nm. Derartige Glasflakes sind kommerziell auf breiter Basis verfügbar. Weitere Vorteile bieten dünnere Glasflakes. Dünnere Substrate führen zu einer geringeren Gesamtschichtdicke der erfindungsgemäßen Mehrschichtperlglanzpigmente. So sind ebenfalls bevorzugt Glasflakes, deren mittlere geometrische Dicke in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich 200 nm bis 400 nm liegt.

In einer weiteren Ausführungsform liegt die mittlere geometrische Dicke für natürlichen oder synthetischen Glimmer als zu beschichtendes Substrat bevorzugt in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm.

Beschichtet man plättchenförmige transparente Substrate unterhalb einer mittleren geometrischen Dicke von 50 nm mit hochbrechenden Metalloxiden, so werden extrem bruchempfindliche Mehrschichtperlglanzpigmente erhalten, die schon beim Einarbeiten in das Anwendungsmedium zerbrechen können, was wiederum eine signifikante Herabsetzung des Glanzes bedingt.

Oberhalb einer mittleren geometrischen Substratdicke von 5000 nm können die Mehrschichtperlglanzpigmente insgesamt zu dick werden. Damit geht eine schlechtere spezifische Deckfähigkeit, d.h. abgedeckte Fläche pro Gewichtseinheit an erfindungsgemäßem Mehrschichtperlglanzpigment, einher sowie eine geringere planparallele Orientierung im Anwendungsmedium. Aus einer schlechteren Orientierung wiederum resultiert ein verminderter Glanz.

Die mittlere geometrische Dicke des plättchenförmigen transparenten Substrates wird anhand eines gehärteten Lackfilmes, in dem die Mehrschichtperlglanzpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die geometrische Dicke des plättchenförmigen transparenten Substrates von 100 Mehrschichtperlglanzpigmenten bestimmt und statistisch gemittelt wird.

Bei einer besonders bevorzugten Ausführungsform weisen die erfindungsgemäßen Mehrschichtperlglanzpigmente, die auch als goniochromatische Mehrschichtperlglanzpigmente bezeichnet werden können, basierend auf plättchenförmigen Glasflakes eine optisch wirksame Beschichtung auf, wobei die optisch wirksame Beschichtung

a) eine nicht absorbierende hochbrechende Schicht A mit einem Brechungsindex n $\geq$ 1,8 und vorzugsweise mit einer optischen Schichtdicke in einem Bereich von 30 nm bis 900 nm

b) eine niedrigbrechende Schicht B mit einem Brechungsindex n < 1,8 und vorzugsweise mit einer optischen Schichtdicke in einem Bereich von > 150 nm bis 500 nm

c) eine selektiv oder nicht selektiv absorbierende hochbrechende Schicht C mit einem Brechungsindex n $\geq$ 1,8 und vorzugsweise mit einer optischen Schichtdicke in einem Bereich von 30 nm bis 900 nm auf.

Bei einer ganz besonders bevorzugten Ausführungsform weisen die erfindungsgemäßen Mehrschichtperlglanzpigmente, basierend auf plättchenförmigen Glasflakes, eine optisch wirksame Beschichtung auf, wobei die optisch wirksame Beschichtung

a) eine nicht absorbierende hochbrechende $TiO_2$ umfassende Schicht A mit einem Brechungsindex n $\geq$ 1,8 und vorzugsweise mit einer optischen Schichtdicke in einem Bereich von 30 nm bis 900 nm

b) eine niedrigbrechende $SiO_2$ umfassende Schicht B mit einem Brechungsindex n < 1,8 und vorzugsweise mit einer optischen Schichtdicke in einem Bereich von >150 nm bis 500 nm

c) eine selektiv oder nicht selektiv absorbierende hochbrechende Eisenoxid umfassende Schicht C mit einem Brechungsindex n $\geq$ 1,8 und vorzugsweise mit einer optischen Schichtdicke in einem Bereich von 30 nm bis 900 nm sowie

d) optional eine äußere Schutzschicht D

auf.

Diese vorgenannten Mehrschichtperlglanzpigmente weisen die Kennzahlen $D_{10}$, $D_{50}$, $D_{90}$ aus der Summenhäufigkeitsverteilung der volumengemittelten Größenverteilungsfunktion mit einem Span $\Delta D$ von 0,8 - 1,2 auf, wobei der Span $\Delta D$ gemäß der Formel $\Delta D = (D_{90} - D_{10})/ D_{50}$ berechnet ist.

Derartige mehrschichtige Perlglanzpigmente weisen einen überraschend hohen Glanz sowie ein hohes Chroma und einen starken Farbflop auf.

Gemäß einer bevorzugten Ausführungsform weisen die Mehrschichtperlglanzpigmente keine silberne Interferenzfarbe auf.

Mehrschichtperlglanzpigmente mit silberner Interferenzfarbe und Chromawerten $C^*_{15} \leq 20$ führen nicht zu Mehrschichtperlglanzpigmenten mit starkem Chroma und hohen Farbflop.

Die Mehrschichtperlglanzpigmente können weiterhin mit wenigstens einer äußeren Schutzschicht D versehen sein, die die Licht-, Wetter-, und/ oder chemische Stabilität des Mehrschichtperlglanzpigmentes weiter erhöht. Bei der äußeren Schutzschicht D kann es sich auch um eine Nachbeschichtung handeln, die die Handhabung des Pigments bei Einarbeitung in unterschiedliche Medien erleichtert.

Die äußere Schutzschicht D der erfindungsgemäßen Mehrschichtperlglanzpigmente kann eine oder zwei Metalloxidschichten der Elemente Si, Al oder Ce umfassen bzw. bevorzugt daraus bestehen. Bei einer Variante wird als äußerste Metalloxidschicht eine Siliziumoxidschicht, vorzugsweise $SiO_2$-Schicht, aufgebracht. Besonders bevorzugt ist hierbei eine Reihenfolge, bei der zunächst eine Ceroxidschicht aufgebracht ist, der dann eine $SiO_2$-Schicht folgt, wie in der WO 2006/021386 A1 beschrieben, deren Inhalt hiermit unter Bezugnahme aufgenommen ist.

Die äußere Schutzschicht D kann des Weiteren an der Oberfläche organischchemisch modifiziert sein. Beispielsweise können ein oder mehrere Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Evonik hergestellt und unter dem Handelsnamen "Dynasylan" vertrieben. Weitere Produkte können von der Fa. Momentive (Silquest-Silane) oder von der Fa. Wacker, beispielsweise Standard- und $\alpha$-Silane aus der GENIOSIL-Produktgruppe, bezogen werden.

Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw. MTES), 3-Mercaptopropyltrimethoxysilan (Dynasylan MTMO; Silquest A-189), 3-Glycidoxypropyltrimethoxysilan (Dynasylan GLYMO, Silquest A-187), Tris[3-(trimethoxysilyl)propyl]isocyanurat (Silquest Y-11597), Bis[3-(triethoxysilyl)propyl)]tetrasulfid (Silquest A-1289), Bis[3-(triethoxysilyl)propyldisulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), (Isocyanatomethyl)methyldimethoxysilan,

(Isocyanatomethyl)trimethoxysilan, 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropylmethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Vorzugsweise werden als organofunktionelle Silane 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw. MTES), beta-(3,4-Epoxycyclohexyl)ethyltrimethoxysilan (Silquest A-186), Bis(triethoxysilyl)ethan (Silquest Y-9805), gamma-Isocyanatopropyltrimethoxysitan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), 3-(Triethoxysilyl)propylbernsteinsäureanhydrid (GENIOSIL GF 20), Vinyltrimethoxysilan (GENIOSIL XL 10) und/ oder Vinyltris(2-methoxyethoxy)silan (GENIOSIL GF 58) verwendet.

Es ist aber auch möglich, andere organofunktionelle Silane auf die erfindungsgemäßen Mehrschichtperlglanzpigmente aufzubringen.

Weiterhin lassen sich, beispielsweise kommerziell von Degussa erhältliche, wässrige Vorhydrolysate einsetzen. Hierzu gehören u.a. wässriges Aminosiloxan (Dynasylan Hydrosil 1151), wässriges amino-/ alkylfunktionelles Siloxan (Dynasylan Hydrosil 2627 oder 2909), wässriges diaminofunktionelles Siloxan (Dynasylan Hydrosil 2776), wässriges epoxyfunktionelles Siloxan (Dynasylan Hydrosil 2926), amino-/ alkylfunktionelles Oligosiloxan (Dynasylan 1146), vinyl-/ alkylfunktionelles Oligosiloxan (Dynasylan 6598), oligomeres Vinylsilan (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Mehrschichtperlglanzpigments an verschiedenartige Bindemittel sehr gut geeignet.

Bevorzugt werden hierzu folgende Verbindungen genommen: 3-Aminopropyltrimethoxysilan (Dynasylan AMMO; Silquest A-1110), 3-Aminopropyltriethoxysilan (Dynasylan AMEO), [3-(2-Aminoethyl)-aminopropyl]trimethoxysilan (Dynasylan DAMO, Silquest A-1120), [3-(2-Aminoethyl)-aminopropyl]triethoxysilan, triaminofunktionelles Trimethoxysilan (Silquest A-1130), Bis-(gamma-trimethoxysilylpropyl)amin (Silquest A-1170), N-Ethyl-gammaaminoisobutyltrimethoxysilan (Silquest A-Link 15), N-Phenyl-gamma-aminopropyltrimethoxysilan (Silquest Y-9669), 4-Amino-3,3-dimethylbutyltrimethoxysilan (Silquest A-1637), N-Cyclohexylaminomethylmethyldiethoxysilan (GENIOSIL XL 924), N-Cyclohexylaminomethyltriethoxysilan (GENIOSIL XL 926), N-Phenylaminomethyltrimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

$$R_{(4-z)}Si(X)_z, \qquad (A)$$

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/ oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

An oder auf der Oberfläche der erfindungsgemäßen Mehrschichtperlglanzpigmente können neben den erwähnten Silanen bzw. Silangemischen auch weitere organisch-chemische Modifizierungsmittel, wie beispielsweise substituierte oder nicht substituierte Alkylreste, Polyether, Thioether, Siloxane, etc. und Mischungen davon angeordnet sein. Es können aber auch anorganisch-chemische Modifizierungsmittel (z.B. $Al_2O_3$ oder $ZrO_2$ oder deren Gemische), welche z.B. die Dispergierbarkeit und/ oder Verträglichkeit im jeweiligen Anwendungsmedium erhöhen können, auf die Pigmentoberfläche aufgebracht werden.

Über die Oberflächenmodifizierung kann beispielsweise die Hydrophille oder Hydrophobie der Pigmentoberfläche verändert und/ oder eingestellt werden. Beispielsweise können über die Oberflächenmodifizierung die Leafing- bzw. Nonleafing-Eigenschaften der erfindungsgemäßen Mehrschichtperlgtanzpigmente verändert und/ oder eingestellt werden. Unter Leafing wird verstanden, dass sich die erfindungsgemäßen Mehrschichtperlglanzpigmente in einem Anwendungsmedium, beispielsweise einem Lack oder einer Druckfarbe, an oder in der Nähe der Grenz- oder Oberfläche des Anwendungsmediums anordnen.

Die Oberflächenmodifizierungsmittel können auch reaktive chemische Gruppen, wie beispielsweise Acrylat-, Methacrylat-, Vinyl-, Isocyanat-, Cyano-, Epoxy-, Hydroxy-, Aminogruppen oder Mischungen davon, aufweisen. Diese chemisch reaktiven Gruppen ermöglichen eine chemische Anbindung, insbesondere Ausbildung von kovalenten Bindungen, an das Anwendungsmedium oder Komponenten des Anwendungsmediums, wie beispielsweise Bindemittel. Hierdurch können beispielsweise die chemischen und/ oder physikalischen Eigenschaften von ausgehärteten Lacken, Farben oder Druckfarben wie Beständigkeit gegenüber Umwelteinflüssen wie Feuchtigkeit, Sonneneinstrahlung, UV-Beständigkeit, etc., oder gegenüber mechanischen Einflüssen, beispielsweise Kratzern etc., verbessert werden.

Die chemische Reaktion zwischen den chemisch-reaktiven Gruppen und dem Anwendungsmedium bzw. Komponenten des Anwendungsmediums kann beispielsweise durch Einstrahlung von Energie, beispielsweise in Form von UV-Strahlung und/ oder Wärme, induziert werden.

Für die Einarbeitung von mit Silanen nachbeschichteten bzw. mit einer äußeren Schutzschicht versehenen Mehrschichtperlglanzpigmenten in kosmetischen Formulierungen muss darauf geachtet werden, dass das entsprechende Silan bzw. das Material der äußeren Schutzschicht nach der Kosmetikverordnung zulässig ist.

Ein erfindungsgemäßes Verfahren zum Herstellen der Mehrschichtperlglanzpigmente umfasst folgende Schritte:

(i) Größenklassieren der zu beschichtenden plättchenförmigen Substrate, so dass die zu beschichtenden plättchenförmigen Substrate eine volumengemittelte Größenverteilungsfunktion mit den Kennzahlen $D_{10}$, $D_{50}$. $D_{90}$ und einem Span $\Delta D$ in einem Bereich von 0,7 - 1,4 aufweisen, wobei der Span $\Delta D$ gemäß der Formel $\Delta D = (D_{90} - D_{10})/ D_{50}$ definiert ist,
(ii) Aufbringen wenigstens der Schichten A bis C auf die plättchenförmigen Substrate sowie optional wenigstens einer Schicht D,
oder
(iii) Aufbringen wenigstens der Schichten A bis C auf die plättchenförmigen Substrate sowie optional wenigstens einer Schicht D,
(iv) Größenklassieren der zu beschichtenden plättchenförmigen Substrate, so dass die zu beschichtenden plättchenförmigen Substrate eine volumengemittelte Größenverteilungsfunktion mit den Kennzahlen $D_{10}$, $D_{50}$, $D_{90}$ und einen Span $\Delta D$ in einem Bereich von 0,7 - 1,4 aufweisen, wobei der Span $\Delta D$ gemäß der Formel $\Delta D = (D_{90} - D_{10}) / D_{50}$ definiert ist.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Perlglanzpigmente die erfindungsgemäße Teilchengrößenverteilung aufweisen.

Hierbei wird der Größenverteilungsspan der Substrate vermessen und ein Endwert eingestellt, der gleich oder kleiner ist als der anvisierte Spanwert der mehrschichtigen Perlglanzpigmente.

Ein enger Span $\Delta D$ der Substrate kann durch geeignete Zerkleinerungs- und/ oder Klassierungsprozesse der zu beschichtenden, künstlichen Substrate erreicht werden. Die zu beschichtenden künstlichen Substrate können beispielsweise durch Kugelmühle, Strahl- oder Rührwerkskugelmühle, Kollergang oder Dissolver zerkleinert werden. Der Span $\Delta D$ der Endfraktion kann durch geeignete Klassierung, wie beispielsweise eine mehrfache Nasssiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw.

Die Metalloxidschichten werden vorzugsweise nasschemisch aufgebracht, wobei die zur Herstellung von Perlglanzpigmenten entwickelten nasschemischen Beschichtungsverfahren angewendet werden können. Bei der Nassbeschichtung werden die Substratpartikel in Wasser suspendiert und mit einem oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf dem zu beschichtenden Substrat aufgefällt wird, ohne dass es zu Nebenfällungen kommt. Der pH-Wert wird üblicherweise durch gleichzeitiges Zudosieren einer Base und/ oder einer Säure konstant gehalten. Anschließend werden die Pigmente abgetrennt, gewaschen und bei 50 - 150°C für 6 -18 Stunden getrocknet und gegebenenfalls 0,5 - 3 Stunden geglüht, wobei die Glühtemperatur im Hinblick auf die jeweils vorliegende Beschichtung optimiert werden kann. In der Regel liegen die Glühtemperaturen zwischen 700 und 1000°C, vorzugsweise zwischen 700 und 900°C. Falls gewünscht können die Pigmente nach Aufbringen einzelner Beschichtungen abgetrennt, getrocknet und gegebenenfalls geglüht werden, um dann zur Auffällung der weiteren Schichten wieder resuspendiert zu werden.

Die Auffällung der $SiO_2$-Schicht auf das zu beschichtende Substrat kann durch Zugabe einer Kalium- oder Natronwasserglaslösung bei einem geeigneten pH-Wert erfolgen. Alternativ kann die $SiO_2$-Schicht über Sol-Gel-Verfahren ausgehend von Alkoxysilanen, wie z.B. Tetraethoxysilanen aufgebracht werden.

Die erfindungsgemäßen Mehrschichtperlglanzpigmente können zur Herstellung von Pigmentpräparationen und Trockenpräparaten eingesetzt werden.

Die erfindungsgemäßen Mehrschichtperlglanzpigmente lassen sich auch vorteilhaft in Abmischungen mit transparenten und deckenden Weiß-, Bunt- und Schwarzpigmenten sowie weiteren Effektpigmenten verwenden.

Weiterhin können die erfindungsgemäßen Mehrschichtperlglanzpigmente beispielsweise in kosmetischen Formulierungen, Kunststoffen, keramischen Materialien, Gläsern und Beschichtungszusammensetzungen wie Farben, Druckfarben, z.B. für den Offset-, Sieb-, Tief-, Flexo-, Sicherheitsdruck oder Bronzierung, Tinten, in Tonem, Lacken, z.B. Autolacken oder Pulverlacken, zur Lasermarkierung von Papier und Kunststoffen, zur Saatguteinfärbung, zur Einfärbung von Lebensmitteln oder pharmazeutischen Erzeugnissen oder zur Farbgebung von (Agrar-)Folien, Zeltplanen oder Textilien verwendet werden.

In kosmetischen Formulierungen können die erfindungsgemäßen Mehrschichtperlglanzpigmente mit für die jeweilige Anwendung geeigneten Roh-, Hilfs- und Wirkstoffen kombiniert werden. Die Konzentration der Mehrschichtperlglanzpigmente in der Formulierung kann zwischen 0,001 Gew.% für Rinse-off-Produkte und 40,0 Gew.-% für Leaveon-Produkte liegen.

Die erfindungsgemäßen Mehrschichtperlglanzpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semipermanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Mehrschichtperlglanzpigmenten weitere Farbmittel und/ oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige der Fa. Eckart), BiOCl-Plättchen, $TiO_2$-Plättchen, Perlglanzpigmente auf der Basis von mit hochbrechenden Metalloxiden beschichtetem synthetischen Glimmer oder basierend auf mit hochbrechenden Metalloxiden beschichteten Glasflakes (wie z.B. die Produktgruppe MIRAGE der Fa. Eckart), $Al_2O_3$-, $SiO_2$- oder $TiO_2$-Plättchen verwendet werden. Außerdem können auch Metalleffektpigmente, wie z.B. die Produktgruppe Visionaire der Fa. Eckart, zugegeben werden. Die Farbmittel können aus anorganischen oder organischen Pigmenten ausgewählt sein.

Im Folgenden wird die Erfindung durch einige Beispiele näher erläutert, ohne auf diese beschränkt zu sein.

**I Herstellung der Pigmente**

**A Klassierung der Substrate**

**Beispiel 1: Klassierung von Glasflakes mit engem Span ΔD = 1,0**

Eine Suspension von 200 g Glasflakes (GF100M der Firma Glassflake Ltd.) in VE-Wasser (VE = vollentsalzt, ca. 3 Gew.-%ig) wurde über ein 100 $\mu$m Sieb klassiert und der Siebdurchgang wiederum über ein 63 $\mu$m Sieb gesiebt. Diese Siebprozedur wurde mit auf dem 63 $\mu$m Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=50 $\mu$m, $D_{50}$=82 $\mu$m, $D_{90}$=132 $\mu$m, Span ΔD = 1,0.

**Vergleichsbeispiel 1: Klassierung von Glasflakes mit breitem Span ΔD = 2,0**

Eine Suspension von 200 g Glasflakes (GF100M der Firma Glassflake Ltd.) in VE-Wasser (ca. 3 Gew.-%ig) wurde über ein 150 $\mu$m Sieb klassiert und der Siebdurchgang wiederum über ein 34 $\mu$m Sieb gesiebt. Diese Siebprozedur wurde mit auf dem 34 $\mu$m Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=25 $\mu$m, $D_{50}$=88 $\mu$m, $D_{90}$=200 $\mu$m, Span ΔD = 1,99.

**B Herstellung einschichtiger Pigmente (Ausgangsmaterial für mehrschichtige Perlglanzpigmente)**

**Vergleichsbeispiel 2 : Herstellung des Ausgangsmaterials für das Beispiel 2**

200 g Glasflakes aus Beispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO2" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**Vergleichsbeispiel 3 : Herstellung des Ausgangsmaterials für das Vergleichsbeispiel 4**

200 g Glasflakes aus Vergleichsbeispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**C Herstellung der Mehrschichtperlglanzpigmente**

**Beispiel 2: Glasflakes/ $TiO_2$(rutil)/ $SiO_2$/ $Fe_2O_3$**

200 g $TiO_2$-beschichtete Glasflakes aus Vergleichsbeispiel 2 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (235 g Wasserglaslösung, 27 Gew.-% $SiO_2$, gemischt mit 235 g VE-Wasser) wurde sodann langsam in die Suspension eingeleitet und der pH-Wert konstant bei pH 7,5 gehalten. Danach wurde noch 20 min nachgerührt, der pH-Wert auf 3,0 gesenkt und sodann eine Lösung von 85 ml FeCl$_3$ (280 g Fe$_2$O$_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein extrem hochglänzendes Mehrschichtpigment mit rötlich-goldener Interferenzfarbe im Glanzwinkel erhalten, die im flachen Winkel zu einem grünlichen Gold abkippt. Das Pigment weist zudem eine rötliche Eigenfarbe auf.

## Vergleichsbeispiel 4: Glasflakes/ TiO$_2$(rutil)/ SiO$_2$/ Fe$_2$O$_3$

200 g TiO$_2$-beschichtete Glasflakes aus Vergleichsbeispiel 3 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (235 g Wasserglaslösung, 27 Gew.-% SiO$_2$, gemischt mit 235 g VE-Wasser) wurde sodann langsam in die Suspension eingeleitet und der pH-Wert konstant bei pH 7,5 gehalten. Danach wurde noch 20 min nachgerührt, der pH-Wert auf 3,0 gesenkt und sodann eine Lösung von 85 ml FeCl$_3$ (280 g Fe$_2$O$_3$/l VE-Wasser) in die Suspension hinzudosiert. Der pH-Wert wurde dabei durch Gegensteuern mit . 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein Mehrschichtpigment mit schwach-rötlicher goldener Interferenzfarbe im Glanzwinkel erhalten, die im flachen Winkel leicht in einen schwachen Goldton abkippt. Das Pigment weist zudem eine rötliche Eigenfarbe auf.

## II Physikalische Charakterisierung

### IIa Winkelabhängige Farbmessungen

Zur Messung der Chromawerte wurden die Mehrschichtperlglanzpigmente mit einer Pigmentierungshöhe von 6 Gew.-% (bezogen auf das Gesamtgewichtes des Nasslacks) in einen konventionellen Nitrocelluloselack (Dr. Renger Erco Bronzemischlack 2615e; Fa. Morton) eingerührt. Dabei wurden die Mehrschichtperlglanzpigmente vorgelegt und anschließend mit einem Pinsel in den Lack dispergiert.
Der fertige Lack wurde auf einem Rakelabzugsgerät (RK Print Coat Instr. LTd. Citenco Abziehgerät Modell K 101) mit einer Nassfilmdicke je nach D$_{50}$-Wert des Mehrschichtperlglanzpigments gemäß Tabelle 2 auf Byk-Gardner Schwarz-Weiß-Rakelkarten (Byko-Chart 2853) appliziert und nachfolgend bei Raumtemperatur getrocknet.
Mit dem Mehrwinkelfarbmessgerät Byk Mac (Fa. Byk Gardener) wurden bei einem konstanten Einfallswinkel von 45° (gemäß Herstellerangaben) bei verschiedenen Beobachtungswinkeln relativ zum Glanzwinkel die L*- und C*-Werte bestimmt. Insbesondere waren die Beobachtungswinkel relativ nahe am Glanzwinkel bei 15° sowie -15° relevant. Als relevanter Chromawert der erfindungsgemäßen Mehrschichtperlglanzpigmente wurde der C*$_{15}$-Wert herangezogen, welcher bei einem 15° vom Glanz entfernten Winkel gemessen wurde.
Stark reflektierende Proben (Idealfall Spiegel) reflektierten nahezu das gesamte eintreffende Licht im sogenannten Glanzwinkel. Hier erschien der Farbeindruck der Interferenzfarbe am stärksten. Je weiter man sich bei der Messung vom Glanzwinkel entfernte, desto weniger Licht und somit Interferenzeffekt konnte gemessen werden.

### IIb Glanzmessungen

Der Glanz ist ein Maß für die gerichtete Reflexion und lässt sich mittels eines Micro-Tri-Gloss-Gerätes charakterisieren. Stärker streuende Proben weisen mithin einen niedrigen Glanz auf.
Es wurden die Nitrolackapplikationen aus IIa mit Hilfe eines Micro-Tri-Gloss Glanzmessgerätes der Fa. Byk Gardner bei einem Messwinkel von 20° für hochglänzende Proben und bei 60° für mittelglänzende Proben auf schwarzem Hintergrund vermessen. Lagen die Glanzwerte bei 60° über 70 Glanzeinheiten, so wurden die Proben bei 20° gemessen (Byk-Gardner Katalog 2007/2008, S. 14).

### IIc Partikelgrößenbestimmung:

Die Größenverteilungskurve wurde mit einem Gerät der Fa. Malvern (Gerät: MALVERN Mastersizer 2000) gemäß Herstellerangaben bestimmt. Hierzu wurden ca. 0,1g des entsprechenden Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der Partikel beinhaltet (Abbildung 1).
Unter der mittleren Größe D$_{50}$ wird im Rahmen dieser Erfindung der D$_{50}$-Wert der Summendurchgangskurve

der Volumengemittelten Größenverteilungsfunktion, wie sie durch Laserbeugungsmethoden erhalten werden, verstanden. Der $D_{50}$-Wert gibt an, dass 50% der Pigmente einen Durchmesser aufweisen, der gleich oder kleiner als der angegebene Wert, beispielsweise 20 $\mu$m, ist.

Entsprechend gibt der $D_{90}$-Wert an, dass 90% der Pigmente einen Durchmesser aufweisen, der gleich oder kleiner des jeweiligen Wertes ist.

Weiterhin gibt der $D_{10}$-Wert an, dass 10% der Pigmente einen Durchmesser aufweisen, der gleich oder kleiner des jeweiligen Wertes ist.

Der Span $\Delta D$, definiert als $\Delta D = (D_{90} - D_{10})/D_{50}$, gibt die Verteilungsbreite wieder.

**III Auswertung:**

Tabelle 3: Charakterisierung der Effektpigmente

| Effektpigment | Aufbau | Glanz, 20° | $C^*_{15}$ | Span |
|---|---|---|---|---|
| Beispiel 2 | Glasflake/$TiO_2$/$SiO_2$/$Fe_2O_3$ | 103,2 | 29,9 | 1,1 |
| Vergleichsbeispiel 4 | Glasflake/$TiO_2$/$SiO_2$/$Fe_2O_3$ | 70,7 | 12,6 | 2,0 |

Anhand der Daten in Tabelle 3 kann man eindeutig erkennen, dass das erfindungsgemäße Beispiel 2 mit dem Schichtaufbau Glasflake/$TiO_2$/$SiO_2$/$Fe_2O_3$ und geringem Span gegenüber dem Vergleichsbeispiel 4 einen signifikant erhöhten Glanz und ein deutlich höheres Chroma im 15°-Winkel aufweist.

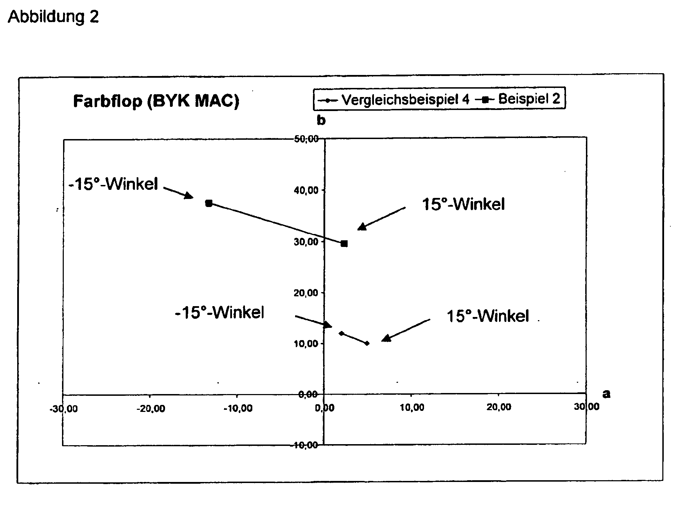

Des Weiteren weist das erfindungsgemäße Beispiel 2 einen deutlich höheren Farbflop auf, wie man der Abbildung 2 entnehmen kann. Hierbei erstreckt sich der Flop vom 1. in den 2. Quadranten des CIELab-Farbkoordinatensystems, während das Vergleichsbeispiel 4 lediglich einen schwachen Flop zeigt.

**IV Anwendungstechnische Beispiele**

In den nachfolgenden kosmetischen Anwendungsbeispielen wurden die erfindungsgemäßen Mehrschichtperlglanzpigmente verwendet, die nach einem der vorstehenden Beispiele hergestellt wurden.

**Beispiel 3:** Transparenter Lippenstift

| INCI Name | Produktname | Gew-% | Lieferant |
|---|---|---|---|
| *Phase A* | | *100.00* | |
| Ethylenediamine/ Hydrogenated Dimer Dilinoleate Copolymer Bis-Di-C14-18 Alkyl Amide | Sylvaclear A2614V | 28.00 | www.arizonachemical.com |
| Bis-Stearyl Ethylenediaminel Neopentyl Glycol/ Hydrogenated Dimer Dilinoleate | Sylvaclear C75V | 28.00 | www.arizonachemical.com |
| Paraffinum Liquidum | Paraffinum Liquidum | 13.80 | www.heess.de |
| Macadamia Integrifolia Seed Oil | Floramac Hawaiian Macadamia Oil-Refined | 10.00 | www.floratech.com |
| Isopropyl Myristate | Isopropyl Myristate | 6.00 | www.vwr.com |
| C12-15 Alkyl Benzoate | Sympatens-LBZ | 6.00 | www.kolb.ch |
| Caprylic/ Capric Triglyceride | Miglyol 812 | 7.00 | www.sasolwax.com |
| Propylparaben | Propyl-4-hydroxybenzoat | 0.20 | www.sigmaaldrich.com |
| *Phase B* | | | |

(fortgesetzt)

| INCI Name | Produktname | Gew-% | Lieferant |
|---|---|---|---|
| **Mehrschichtperlglanzpigment** | **Mehrschichtperlglanzpigment** | **1.00** | |

Das Mehrschichtperlglanzpigment kann in einem Bereich von 0,01 bis 5,0 Gew.-% eingesetzt werden. Der Ausgleich kann mit Paraffinum Liquidum erfolgen.

Phase A wurde auf 85°C erhitzt, danach wurde Phase B der Phase A hinzugegeben und gemischt. Anschließend wurde die Mischung mit einer Temperatur von 75°C in einem Lippenstiftform abgefüllt.

**Beispiel 4:** Creme Lidschatten

| INCI Name | Produktname | Gew-% | Lieferant |
|---|---|---|---|
| *Phase A* | | 100.00 | |
| Castor Oil | Castor Oil | 28.70 | www.riedeldehaen. com |
| Octyl Palmitate | Liponate EHP | 6.00 | www.lipochemicals. com |
| Cocos Nucifera (Coconut) Oil | Lipovol C-76 | 7.00 | www.lipochemicals. com |
| Bees Wax | Ewacera 12 | 6.00 | www.wagnerlanolin .com |
| Isopropyl Lanolate | Ewalan IP | 5.00 | www.wagnerlanolin .com |
| Persea Gratissima (Avocado) Oil and Hydrogenated Avocado Oil | Avocado Butter | 7.00 | www.impag.de |
| Magnesium Stearate | Magnesium Stearate | 3.00 | www.sigmaaldrich.c om |
| Bis-Hydroxyethoxypropyl Dimethicone | Dow Corning 5562 Carbinol Fluid | 7.00 | www.dowcorning.c om |
| Dimethicone/ Vinyl Dimethicone Crosspolymer and Silica | Dow Corning 9701 Cosmetic Powder | 5.00 | www.dowcorning.c om |
| Phenoxyethanol (and) Methylparaben (and) Ethylparaben (and) Butylparaben | Uniphen P-23 | 0.30 | www.induchem.co m |
| *Phase B* | | | |
| **Mehrschichtperlglanzpigment** | **Mehrschichtperlglanzpigment** | **25.00** | |

Das Mehrschichtperlglanzpigment kann in einem Bereich von 5,0 bis 30,0 Gew.-% eingesetzt werden. Der Ausgleich kann mit Castor Oil erfolgen.

Phase A wurde gemischt und auf 85°C erhitzt, Inhaltsstoffe von Phase B wurden ebenfalls zusammengemischt und anschließend unter Rühren zu Phase A hinzugegeben. Nach Abfüllen in ein entsprechendes Behältnis wurde die Mischung auf Raumtemperatur abgekühlt.

**Beispiel 5:** Duschgel

| INCI Name | Produktname | Gew-% | Lieferant |
|---|---|---|---|
| ***Phase A*** | | *100.00* | |
| **Mehrschichtperlglanzpigment** | **Mehrschichtperlglanzpigment** | **0.01** | |
| Aqua | Wasser | 68.21 | |

(fortgesetzt)

| INCI Name | Produktname | Gew-% | Lieferant |
|---|---|---|---|
| Blue 1 (0.5 Gew.-% aqueous solution) | Blue 1 | 0.10 | www.sunchemicals.com |
| Acrylates/ C 10-30 Alkyl Acrylate Crosspolymer | Carbopol ETD 2020 | 1.00 | www.noveon.com |
| Propylene Glycol | 1, 2- Propanediol | 1.00 | www.vwr.com |
| *Phase B* | | | |
| TEA- Lauryl Sulfate | Texapon T 42 | 22.00 | www.cognis.com |
| Cocamide DEA | Rewomid DC 212 S | 3.00 | www.degussa.com |
| Cocamidopropyl Betaine | Tego Betain F 50 | 4.00 | www.cognis.com |
| Disodium EDTA | Edeta BD | 0.05 | www.basf.com |
| *Phase C* | | | |
| Triethanolamine | Triethanolamine | 0.30 | www.vwr.com |
| Phenoxyethanol, Ethylhexylglycerin | Euxyl PE 9010 | 0.60 | www.schuelke.com |

Das Mehrschichtperlglanzpigment kann in einem Bereich von 0,01 bis 1,0 Gew.-% eingesetzt werden. Der Ausgleich kann mit Wasser erfolgen.

Carbopol wurde in Phase A dispergiert und 15 Minuten gerührt und auf 65°C erhitzt. Danach wurden die Rohstoffe der Phase B einzeln zu Phase A unter langsamem Rühren hinzugefügt. Anschließend wurde die Mischung auf 40°C abgekühlt und Phase C zugegeben.

**Beispiel 6:** Gepresster Lidschatten

| INCI Name | Produktname | Gew-% | Lieferant |
|---|---|---|---|
| *Phase A* | | *100.00* | |
| Mica | Silk Mica | 17.00 | www.vwr.com |
| Boron Nitride | Softouch CCS 102 | 2.50 | www.advceramicsc os.com |
| Zinc Stearate | Kemilub EZ-V | 7.00 | www.undesa.com |
| Talc | Talc Powder | 38.50 | www.riedeldehaen. com |
| **Mehrschichtperlglanzpigment** | **Mehrschichtperlglanzpigment** | **25.00** | |
| *Phase B* | | | |
| Dimethicone | Dow Corning 200 Fluid 5 cst. | 5.00 | www.dowcorning.c om |
| Cyclomethicone (and) Dimethicone Crosspolymer | Dow Corning 9040 Elastomer | 5.00 | www.dowcorning.c om |

Das Mehrschichtperlglanzpigment kann in einem Bereich von 5,0 bis 40,0 Gew.-% eingesetzt werden. Der Ausgleich kann mit Talc erfolgen.

Phase A wurde für 30s bei 2500 rpm in einem Hochgeschwindigkeitsmixer gemischt. Anschließend wurde Phase B zugegeben und die Mischung für 60s bei 3000 rpm im gleichen Mixer gemischt. Zuletzt wurde die Pulvermischung mittels einer Lidschattenpresse bei 150 bar für 30s in Form gepresst.

**Beispiel 7:** Lip Gloss

| INCI Name | Produktname | Gew-% | Lieferant |
|---|---|---|---|
| *Phase A* | | *100.00* | |
| Hydrogenated Polyisobutene (and) Ethylene/Propylene/Styrene Copolymer (and) Butylene/Ethylene/Styrene Copolymer | Versagel ME 750 | 78.90 | www.penreco. com |
| Simmondsia Chinensis (Jojoba) Seed Oil | Jojoba Oil - Natural/Golden | 2.00 | www.biochemica.co m |
| Caprylyl Trimethicone | Silcare Silicone 31M50 | 7.00 | www.clariant.com |
| Stearyl Dimethicone | Silcare Silicone 41 M65 | 3.20 | www.clariant.com |
| Hydrogenated Polydecene | Nexbase 2002 | 4.00 | www.jandekker. com |
| Isopropyl Myristate | Isopropyl Myristate | 4.50 | www.vwr.com |
| *Phase B* | | | |
| **Mehrschichtpertglanzpigment** | **Mehrschichtperlglanzpigment** | **0.20** | |
| Propylparaben | Propyl-4-hydroxybenzoat | 0.20 | www. sigmaaldrich.com |

Das Mehrschichtperlglanzpigment kann in einem Bereich von 0,01 bis 0,50 Gew.-% eingesetzt werden. Der Ausgleich kann mit Versagel ME 750 erfolgen.

Phase A wurde auf 85°C erhitzt, anschließend wurden die Inhaltstoffe der Phase B einzeln zu Phase A hinzugegeben und gerührt, bis eine gleichmäßige Konsistenz entstand, und dann in ein Lip Gloss Gefäß abgefüllt.

**Beispiel 8:** Nagellack

| INCI Name | Produktname | Gew-% | Lieferant |
|---|---|---|---|
| *Phase* A | | *100.00* | |
| **Mehrschichtperlglanz pigment** | **Mehrschichtperlglanzpigment** | **2.00** | |
| *Phase B* | | | |
| Butylacetat (and) Ethylacetat (and) Nitrocellulose (and) Isopropyl Alcohol | International Lacquers Nailpolish & Care Base 359 | 98.00 | www.Internationallacqu ers.lu |

Das Mehrschichtperlglanzpigment kann in einem Bereich von 0,1 bis 10,0 Gew.-% eingesetzt werden. Der Ausgleich kann mit International Lacquers Nailpolish erfolgen.

Phase A und Phase B wurden gemischt und anschließend in ein angemessenes Behältnis abgefüllt.

**Patentansprüche**

1. Mehrschichtperlglanzpigmente basierend auf plättchenförmigen transparenten Substraten mit optisch wirksamer Beschichtung,
**dadurch gekennzeichnet,**
**dass** die optisch wirksame Beschichtung wenigstens

a) eine nicht absorbierende hochbrechende Schicht A mit einem Brechungsindex n ≥ 1,8
b) eine niedrigbrechende Schicht B mit einem Brechungsindex n < 1,8 mit einer optischen Schichtdicke > 150 nm

c) eine absorbierende hochbrechende Schicht C mit einem Brechungsindex n ≥ 1,8
sowie

d) optional eine äußere Schutzschicht D

umfasst, und dass die Mehrschichtperlglanzpigmente eine Summenhäufigkeitsverteilung der volumengemittelten Größenverteilungsfunktion mit den Kennzahlen $D_{10}$, $D_{50}$, $D_{90}$ und einem Span $\Delta D$ von 0,7 -1,4 aufweisen, wobei der Span $\Delta D$ gemäß der Formel $\Delta D = (D_{90} - D_{10})/ D_{50}$ berechnet ist.

2. Mehrschichtperlglanzpigmente nach Anspruch 1,
**dadurch gekennzeichnet,**
**dass** die Mehrschichtperlglanzpigmente keine silberne Interferenzfarbe aufweisen.

3. Mehrschichtperlglanzpigmente nach einem der vorstehenden Ansprüche,
**dadurch gekennzeichnet,**
**dass** die Mehrschichtperlglanzpigmente einen Span $\Delta D$ von 0,7 bis 1,3 aufweisen.

4. Mehrschichtperlglanzpigmente nach einem der vorstehenden Ansprüche,
**dadurch gekennzeichnet,**
**dass** die optische Schichtdicke der Schicht A in einem Bereich von 30 nm bis 900 nm liegt.

5. Mehrschichtperlglanzpigmente nach einem der vorstehenden Ansprüche,
**dadurch gekennzeichnet,**
**dass** die optische Schichtdicke der Schicht B in einem Bereich von > 150 nm bis 500 nm liegt.

6. Mehrschichtperlglanzpigmente nach einem der vorstehenden Ansprüche,
**dadurch gekennzeichnet,**
**dass** die Schicht C ein Metalloxid oder ein Metallsulfid umfasst.

7. Mehrschichtperlglanzpigmente nach einem der vorstehenden Ansprüche,
**dadurch gekennzeichnet,**
**dass** die optische Schichtdicke der Schicht C in einem Bereich von 30 nm bis 900 nm liegt.

8. Mehrschichtperlglanzpigmente nach einem der vorstehenden Ansprüche,
**dadurch gekennzeichnet,**
**dass** die Schicht A Titanoxid, vorzugsweise Titandioxid, umfasst.

9. Mehrschichtperlglanzpigmente nach einem der vorstehenden Ansprüche,
**dadurch gekennzeichnet,**
**dass** die Schicht B Siliziumoxid, bevorzugt $SiO_2$ umfasst.

10. Mehrschichtperlglanzpigmente nach einem der vorstehenden Ansprüche,
**dadurch gekennzeichnet,**
**dass** die Schicht C absorbierendes Eisenoxid oder Mischungen verschiedener
Eisenoxide, von denen wenigstens ein Eisenoxid absorbierendes Eisenoxid ist, umfasst.

11. Mehrschichtperlglanzpigmente nach einem der vorstehenden Ansprüche,
**dadurch gekennzeichnet,**
**dass** die plättchenförmigen transparenten Substrate der Mehrschichtperlglanzpigmente aus der Gruppe, bestehend aus natürlichem Glimmer, synthetischem Glimmer, Glasflakes, $SiO_2$-Plättchen, Al₂O3-Plättchen und deren Gemische, ausgewählt sind.

12. Verfahren zum Herstellen der Mehrschichtperlglanzpigmente nach einem der vorstehenden Ansprüche,
**dadurch gekennzeichnet,**
**dass** es folgende Schritte umfasst:

(i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 - 1,4 aufweisen, wobei der

Span $\Delta D$ gemäß der Formel $\Delta D = (D_{90} - D_{10})/ D_{50}$ definiert ist,

(ii) Aufbringen wenigstens der Schichten A bis C auf die plättchenförmigen transparenten Substrate sowie optional wenigstens einer Schicht D,

oder

(iii) Aufbringen wenigstens der Schichten A bis C auf die plättchenförmigen transparenten Substrate sowie optional wenigstens einer Schicht D,

(iv) Größenklassieren der zu beschichtenden plättchenförmigen transparenten Substrate, so dass die zu beschichtenden plättchenförmigen Substrate eine volumengemittelte Größenverteilungsfunktion mit den Kennzahlen $D_{10}$, $D_{50}$, $D_{90}$ und einen Span $\Delta D$ in einem Bereich von 0,7 - 1,4 aufweisen, wobei der Span $\Delta D$ gemäß Formel $\Delta D = (D_{90} - D_{10})/ D_{50}$ definiert ist.

**13.** Verwendung der Mehrschichtperlglanzpigmente nach einem der Ansprüche 1 bis 11 in kosmetischen Formulierungen, Kunststoffen, Folien, Textilien, keramischen Materialien, Gläsern und Beschichtungszusammensetzungen wie Farben, Druckfarben, Lacken, Pulverlacken oder Elektrotauchlacken.

**14.** Formulierung enthaltend Mehrschichtperlglanzpigmente nach einem der Ansprüche 1 bis 11.

**15.** Gegenstand,
**dadurch gekennzeichnet,**
**dass** der Gegenstand die Mehrschichtperlglanzpigmente nach einem der Ansprüche 1 bis 11 aufweist oder enthält.

**Claims**

**1.** Multi-layered pearlescent pigments based on platelet-shaped, transparent substrates with an optically active coating, **characterised in that** the optically active coating comprises at least

a) a non-absorbing, high-index layer A with a refractive index $n \geq 1.8$,
b) a low-index layer B with a refractive index $n < 1.8$ with an optical layer thickness $> 150$ nm,
c) an absorbing, high-index layer C with a refractive index $n \geq 1.8$,
and
d) optionally an outer protective layer D,

and the multi-layered pearlescent pigments have a cumulative frequency distribution of the volume-averaged size distribution function with the indices $D^{10}$, $D^{50}$ and $D^{90}$ and a span $\Delta D$ of 0.7 - 1,4, the span $\Delta D$ being calculated in accordance with the equation $\Delta D = (D^{90}-D^{10})/D^{50}$.

**2.** Multi-layered pearlescent pigments as claimed in claim 1,
**characterised in that**
the multi-layered pearlescent pigments do not contain any silver interference colour.

**3.** Multi-layered pearlescent pigments as claimed in one of the preceding claims,
**characterised in that**
the multi-layered pearlescent pigments have a span $\Delta D$ of 0.7 to 1.3.

**4.** Multi-layered pearlescent pigments as claimed in one of the preceding claims,
**characterised in that**
the optical layer thickness of layer A lies in a range of 30 nm to 900 nm.

**5.** Multi-layered pearlescent pigments as claimed in one of the preceding claims,
**characterised in that**
the optical layer thickness of layer B lies in a range of $> 150$ nm to 500 nm.

**6.** Multi-layered pearlescent pigments as claimed in one of the preceding claims,
**characterised in that**
layer C comprises a metal oxide or a metal sulphide.

**7.** Multi-layered pearlescent pigments as claimed in one of the preceding claims,
**characterised in that**
the optical layer thickness of layer C lies in a range of 30 nm to 900 nm.

**8.** Multi-layered pearlescent pigments as claimed in one of the preceding claims,
**characterised in that**
layer A comprises titanium oxide, preferably titanium dioxide.

**9.** Multi-layered pearlescent pigments as claimed in one of the preceding claims,
**characterised in that**
layer B comprises silicon oxide, preferably $SiO_2$.

**10.** Multi-layered pearlescent pigments as claimed in one of the preceding claims,
**characterised in that**
layer C comprises absorbing iron oxide or mixtures of different iron oxides, of which at least one iron oxide is absorbing iron oxide.

**11.** Multi-layered pearlescent pigments as claimed in one of the preceding claims,
**characterised in that**
the platelet-shaped, transparent substrates of the multi-layered pearlescent pigments are selected from the group comprising natural mica, synthetic mica, glass flakes, $SiO_2$ platelets, $Al_2O_3$ platelets and mixtures thereof.

**12.** Method of producing multi-layered pearlescent pigments as claimed in one of the preceding claims,
**characterised in that**
it comprises the following steps:

(i) classifying the size of the platelet-shaped, transparent substrates to be coated so that the platelet-shaped, transparent substrates to be coated have a volume-averaged size distribution function with the indices $D^{10}$, $D^{50}$, $D^{90}$ and a span $\Delta D$ in a range of 0.7 - 1.4, the span $\Delta D$ being defined by the equation $\Delta D = (D^{90} - D^{10})/D^{50}$,
(ii) applying at least layers A to C to the platelet-shaped, transparent substrates and optionally at least a layer D
or
(iii) applying at least layers A to C to the platelet-shaped, transparent substrates and optionally at least a layer D,
(iv) classifying the size of the platelet-shaped, transparent substrates to be coated so that the platelet-shaped, transparent substrates to be coated have a volume-averaged size distribution function with the indices $D^{10}$, $D^{50}$, $D^{90}$ and a span $\Delta D$ in a range of 0.7 - 1.4, the span $\Delta D$ being defined by the equation $\Delta D = (D^{90} - D^{10})/D^{50}$.

**13.** Use of the multi-layered pearlescent pigments as claimed in one of claims 1 to 11 in cosmetic formulations, plastics, foils, textiles, ceramic materials, glasses and coating compositions such as paints, printing inks, varnishes, powder coatings or electro-deposition coatings.

**14.** Formulation containing multi-layered pearlescent pigments as claimed in one of claims 1 to 11.

**15.** Object
**characterised in that**
the object has or contains the multi-layered pearlescent pigments as claimed in one of claims 1 to 11.


**Revendications**

**1.** Pigments perlés multicouches à base de substrats transparents en forme de plaquettes à revêtement optiquement actif, **caractérisés en ce que** le revêtement optiquement actif comprend

a) une couche A hautement réfringente non absorbante d'un indice de réfraction n $\geq$ 1,8,
b) une couche B faiblement réfringente d'un indice de réfraction n < 1,8 et d'une épaisseur de couche optique > 150 nm,
c) une couche C hautement réfringente absorbante d'un indice de réfraction n $\geq$ 1,8,
ainsi que
d) éventuellement une couche de protection extérieure D

et **en ce que** les pigments perlés multicouches présentent une répartition des fréquences cumulées de la fonction de distribution de taille moyennée en volume qui présente les caractéristiques $D_{10}$, $D_{50}$, $D_{90}$ et un intervalle $\Delta D$ de 0,7 à 1,4, l'intervalle $\Delta D$ étant calculé selon la formule $\Delta D = (D^{90}-D^{10})/D_{50}$.

2. Pigments perlés multicouches selon la revendication 1, **caractérisés en ce que** les pigments perlés multicouches ne comprennent pas de couleur interférentielle argentée.

3. Pigments perlés multicouches selon l'une quelconque des revendications précédentes, **caractérisés en ce que** les pigments perlés multicouches présentent un intervalle $\Delta D$ de 0,7 à 1,3.

4. Pigments perlés multicouches selon l'une quelconque des revendications précédentes, **caractérisés en ce que** l'épaisseur de couche optique de la couche A se situe dans une plage allant de 30 nm à 900 nm.

5. Pigments perlés multicouches selon l'une quelconque des revendications précédentes, **caractérisés en ce que** l'épaisseur de couche optique de la couche B se situe dans une plage allant de > 150 nm à 500 nm.

6. Pigments perlés multicouches selon l'une quelconque des revendications précédentes, **caractérisés en ce que** la couche C comprend un oxyde métallique ou un sulfure métallique.

7. Pigments perlés multicouches selon l'une quelconque des revendications précédentes, **caractérisés en ce que** l'épaisseur de couche optique de la couche C se situe dans une plage allant de 30 nm à 900 nm.

8. Pigments perlés multicouches selon l'une quelconque des revendications précédentes, **caractérisés en ce que** la couche A comprend de l'oxyde de titane, de préférence du dioxyde de titane.

9. Pigments perlés multicouches selon l'une quelconque des revendications précédentes, **caractérisés en ce que** la couche B comprend de l'oxyde de silicium, de préférence $SiO_2$.

10. Pigments perlés multicouches selon l'une quelconque des revendications précédentes, **caractérisés en ce que** la couche C comprend de l'oxyde de fer absorbant ou des mélanges de différents oxydes de fer, parmi lesquels au moins un oxyde de fer est un oxyde de fer absorbant.

11. Pigments perlés multicouches selon l'une quelconque des revendications précédentes, **caractérisés en ce que** les substrats transparents en forme de plaquettes des pigments perlés multicouches sont choisis dans le groupe constitué par le mica naturel, le mica synthétique, les flocons de verre, les plaquettes de $SiO_2$, les plaquettes d'$Al_2O_3$ et leurs mélanges.

12. Procédé de fabrication des pigments perlés multicouches selon l'une quelconque des revendications précédentes, **caractérisé en ce qu'**il comprend les étapes suivantes :

   (i) la classification grossière des substrats transparents en forme de plaquettes à revêtir de manière à ce que les substrats transparents en forme de plaquettes à revêtir présentent une fonction de distribution de taille moyennée en volume présentant les caractéristiques $D_{10}$, $D_{50}$, $D_{90}$ et un intervalle $\Delta D$ dans une plage allant de 0,7 à 1,4, l'intervalle $\Delta D$ étant calculé selon la formule $\Delta D = (D_{90}-D_{10})/D_{50}$,
   (ii) l'application d'au moins les couches A à C sur les substrats transparents en forme de plaquettes, ainsi qu'éventuellement d'au moins une couche D,
   ou
   (iii) l'application d'au moins les couches A à C sur les substrats transparents en forme de plaquettes, ainsi qu'éventuellement d'au moins une couche D,
   (iv) la classification grossière des substrats transparents en forme de plaquettes à revêtir de manière à ce que les substrats transparents en forme de plaquettes à revêtir présentent une fonction de distribution de taille moyennée en volume présentant les caractéristiques $D_{10}$, $D_{50}$, $D_{90}$ et un intervalle $\Delta D$ dans une plage allant de 0,7 à 1,4, l'intervalle $\Delta D$ étant calculé selon la formule $\Delta D = (D_{90}-D_{10})/D_{50}$.

13. Utilisation des pigments perlés multicouches selon l'une quelconque des revendications 1 à 11 dans des formulations cosmétiques, des plastiques, des feuilles, des textiles, des matériaux céramiques, des verres et des compositions de revêtement telles que des peintures, des encres d'impression, des laques, des laques en poudre ou des peintures à électrodéposition.

**14.** Formulation contenant des pigments perlés multicouches selon l'une quelconque des revendications 1 à 11.

**15.** Article, **caractérisé en ce que** l'article comprend ou contient les pigments perlés multicouches selon l'une quelconque des revendications 1 à 11.

Abbildung 1

Beeinflussung der Laserbeugung durch Partikeleigenschaften

Partikeleigenschaften:

d: Durchmesser

1: Beugung

2: Brechung

3: Reflexion

4: Absorption

Abbildung 2

Farbflop (BYK MAC) — Vergleichsbeispiel 4 —■— Beispiel 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 07246366 B
- DE 19525503 A1
- DE 19953655 A1
- US 6875264 B2
- EP 1474486 A2
- WO 2006110359 A2
- US 20060042509 A1
- WO 2009103322 A1
- WO 03006558 A2
- EP 0289240 A1
- WO 2004056716 A1
- WO 2005063637 A1
- EP 1980594 B1
- WO 2006021386 A1